# EUROPEAN PATENT SPECIFICATION

(11) **EP 3 225 686 B1**
(45) Date of publication and mention of the grant of the patent: **31.07.2019**
(21) Application number: 17163622.8
(22) Date of filing: 29.03.2017
(51) Int. Cl.: C12Q 1/6806, C12Q 1/6832, C12N 9/22, C12N 15/63

(54) **NOVEL HOMING ENDONUCLEASE DERIVED FROM ARABIDOPSIS THALIANA**
NEUARTIGE, AUS ARABIDOPSIS THALIANA STAMMENDE HOMING-ENDONUKLEASE
NOUVELLE ENDONUCLÉASE DE HOMING DÉRIVÉE D'ARABIDOPSIS THALIANA

(30) Priority: 30.03.2016 KR 20160038554
(43) Date of publication of application: 04.10.2017
(73) Proprietor: Industry-Academic Cooperation Foundation Yonsei University, Seoul 03722 (KR)
(72) Inventor: YANG, Seong Wook, Seoul 06270 (KR); CHO, Seok Keun, Gyeonggi-do 10492 (KR); RYU, Moon Young, Gyeonggi-do 10492 (KR)
(74) Representative: Botti, Mario

(56) References cited:
- US-A1- 2009 019 601
- UniProtKB O22931_ARATH; ROUNSLEY S.D. et al.: "At2g30350 - Putative uncharacterized protein At2g30350 - Arabidopsis thaliana (Mouse-ear cress) - At2g30350 gene & protein", , 1 January 1998 (1998-01-01), XP055394320, Retrieved from the Internet: URL:http://www.uniprot.org/uniprot/O22931 [retrieved on 2017-07-27]
- BENJAMIN P KLEINSTIVER ET AL: "Monomeric site-specific nucleases for genome editing", PNAS, PROCEEDINGS OF THE NATIONAL ACADEMY OF SCIENCES,, vol. 109, no. 21, 22 May 2012 (2012-05-22) , pages 8061-8066, XP002691036, ISSN: 1091-6490, DOI: 10.1073/PNAS.1117984109 [retrieved on 2012-05-07]

## Description

### CROSS-REFERENCE TO RELATED APPLICATION

This application claims priority to and the benefit of Korean Patent Application No. 2016-0038554, filed on March 30, 2016.

### BACKGROUND

### 1. Field of the Invention

The present invention relates to the use of a novel GIY-YIG homing endonuclease isolated from *Arabidopsis thaliana.*

### 2. Discussion of Related Art

In recent years, microRNA (miR, miRNA) has emerged as an important novel class of regulatory RNA involved in a wide range of biological processes. Small non-coding RNA molecules have an influence on protein expression patterns by promoting RNA degradation, repressing mRNA translation, and regulating gene transcription.

Micro-RNA (miRNA) is a single-stranded short (21 to 22 nucleotides) non-coding RNA molecule. The miRNA has an important role in post-translational gene regulation. The miRNA forms a complementary base pair (base pairing) and induces messenger RNA (mRNA) degradation or translational repression.

Over 2,500 miRNAs have been identified in the human genome (miRBase, reported articles). Most of the miRNAs may target one or more mRNAs at the same time. Also, different miRNAs may target the same mRNA, and may induce a synergistic effect. The miRNA plays an important role in various cellular processes such as differentiation, proliferation, apoptosis, metabolic homeostasis, oncogenesis, and DNA methylation (Li H, et al., Hypertension 2003; 42(5): 895-900). Therefore, the diagnosis and analysis of miRNA expression may provide important information on physiological and pathological conditions, and may be used to diagnose genetic or infectious diseases so as to develop a method of treating the diseases.

As a method of detecting complementary nucleic acid strands included in a certain sample, a method of hybridizing a labeled probe and nucleic acid has been used.

Northern and Southern blotting are the most widely used methods of detecting nucleic acids using the above principle, and analytical methods such as fluorescence *in situ* hybridization (FISH), polymerase chain reaction (PCR), and microarray analysis are also based on a sequence-specific reaction through Watson-Crick base pairing.

However, a method of detecting a nucleic acid based on the sequence-specific reaction may cause false positives due to non-specific hybridization, and requires additional long-term processes such as the Northern or Southern blot to determine the false positives.

Since nucleic acids, particularly genomic DNAs have an influence on the viscosity in a sample or interfere with purification, downstream analysis, or application, the nucleic acids may be a barrier to purification and analysis of cell cultures, cell lysates, proteins, etc.

Therefore, the nucleic acids may be physically, chemically, or enzymatically removed, and the enzymatic removal may be performed using nucleic acid hydrolases. Benzonase, OmniCleave, or DNase I has been used to enzymatically remove the nucleic acids before the purification and analysis of the cell lysates and proteins.

For example, a nucleic acid amplification technique such as PCR is a biotechnologically powerful tool that may prepare many copies of a target sequence in a sample containing a small amount of nucleic acids.

A PCR reaction is carried out by adding a primer complementary to a single strand of a double-stranded target sequence to a reaction mixture including a target sequence and free nucleotides. In this case, the PCR reaction mixture may be treated with an endonuclease cleaving an internal bond of the target sequence to prevent DNA amplification which causes false positives.

However, there are different types of endonucleases, each of which has a different activity or site of action.

For example, endonuclease I is a periplasmic or extracellular monomeric enzyme having a molecular weight of approximately 25 kDa which cleaves RNA and DNA in a sequence-dependent fashion. The enzyme is found in various proteobacteria and Fibrobacters. The enzyme structurally has a mixed α/β topology including nine β strands, five short helixes, and five long helixes. The enzyme may cleave plasmids and ssDNA, and may also cleave the 3' terminus of a phosphodiester bond.

Meanwhile, GIY-YIG homing endonucleases may cleave a single-stranded 5'-flap of DNA having a Y-shaped junction structure, and a loop structure of hairpin DNA, but exhibit no cleavage activity against RNA. Accordingly, the GIY-YIG homing endonucleases may not remove target-like miRNA which may cause false positives during miRNA detection in spite of the construct-specific activity, and may not be applied to diagnostic and analytical methods using miRNA as a target.

US 2009/0019601 discloses recombinant polypeptides useful for improvements of plants.

The present inventors have conducted long-term research to find a novel enzyme having a bifunctional nature based on RNA and DNA, and sought to make industrial use of the novel enzyme.

### SUMMARY OF THE INVENTION

Therefore, the present invention is designed to solve the problems of the prior art, and it is the goal of the present invention to provide a novel enzyme which has a bifunctional nature based on RNA and DNA and is able to be effectively used during miRNA detection due to construct-specific activity.

According to an aspect of the present invention, there is provided the use of an isolated homing endonuclease (HIGLE) consisting of the amino acid sequence set forth in SEQ ID NO: 1 which (a) includes a GIY-YIG domain, (b) has a bifunctional nature based on RNA and DNA, and (c) exhibits construct-specific activity with respect to 3' and 5' overhangs in the detection of microRNA (miRNA).

According to one exemplary embodiment, the homing endonuclease may be isolated from *Arabidopsis thaliana.*

According to one exemplary embodiment, the GIY-YIG domain may consist of the amino acid sequence set forth in SEQ ID NO: 2.

According to yet another aspect of the present invention, there is provided a method of detecting microRNA (miRNA) comprising treating a miRNA with a homing endonuclease as defined above.

According to one exemplary embodiment, the nucleic acid may be a double-stranded nucleic acid including 3' and 5' overhangs.

According to one exemplary embodiment, the double-stranded nucleic acid may have a bulge structure formed by non-complementary base pairing between base sequences in regions other than the 3' and 5' overhangs.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:

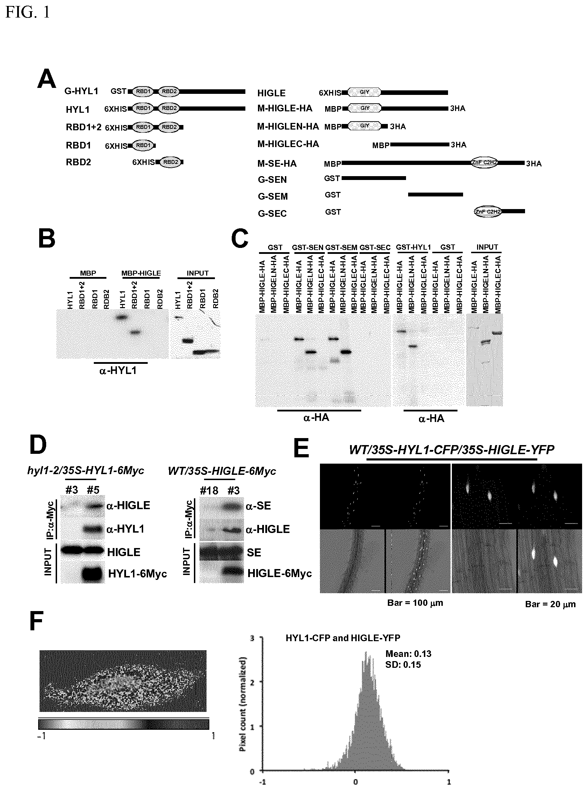

FIG. 1 is a diagram showing the interaction between HIGLE, HYL1 and SE. (A) shows recombinant proteins and deletion variants. (B) shows results of performing a pull-down assay *in vitro.* (C) N-terminal, middle and C-terminal domains of GST-fused SE were used as baits for MBP-HIGLE-HA and the deletion variants. (D) shows results of performing co-immunoprecipitation analysis between HIGLE, HYL1 and SE. (E) shows results of co-expressing HIGLE-YFP and HYL1-CFP in the nuclei from a seedling. (F) shows results of performing FRET analysis using HYL1-CFP and HIGLE-YFP as a donor-acceptor pair;

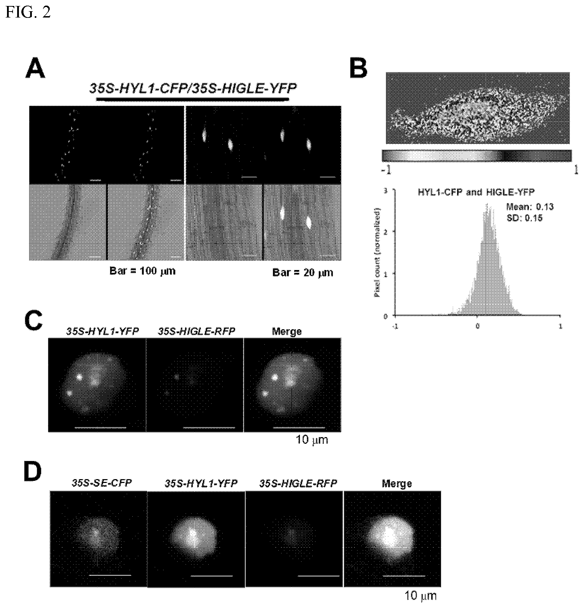

FIG. 2 shows that differently labeled HIGLE, HYL1 and SE partially overlap each other in the nuclei. (A) shows results of co-expressing HIGLE-YFP and HYL1-CFP in the nuclei from a seedling. (B) shows results of performing FRET analysis using HYL1-CFP and HIGLE-YFP as a donor-acceptor pair. (C) shows HIGLE-RFP and HYL1-YFP co-localized in the nuclei from tobacco. (D) shows different emission images of SERRATE-CFP, HYL1-YFP and HIGLE-RFP overlapped on speckles in the tobacco nuclei;

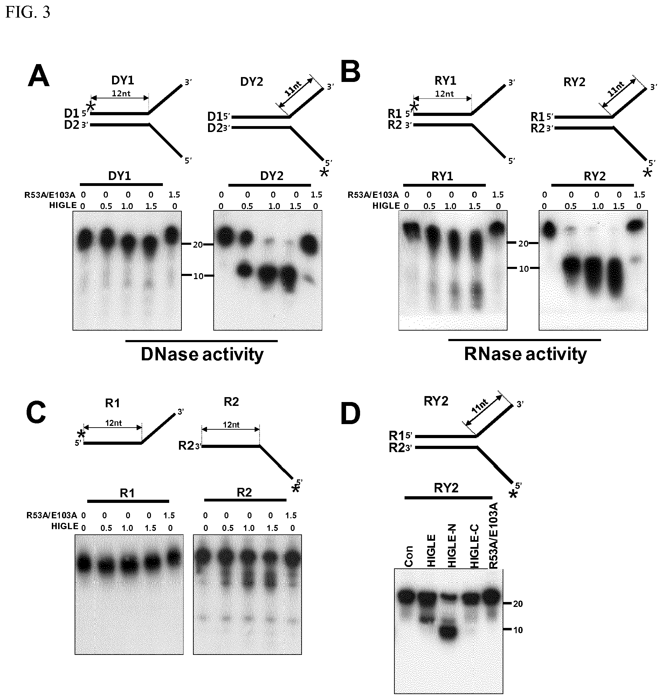

FIG. 3 shows a bifunctional nature of recombinant HIGLE. (A) shows construct-specific deoxyribonuclease (DNase) activity of HIGLE. (B) shows construct-specific ribonuclease (RNase) activity of HIGLE. (C) shows HIGLE having no cleavage activity against single-stranded RNA. (D) shows an identified domain of HIGLE having RNase activity;

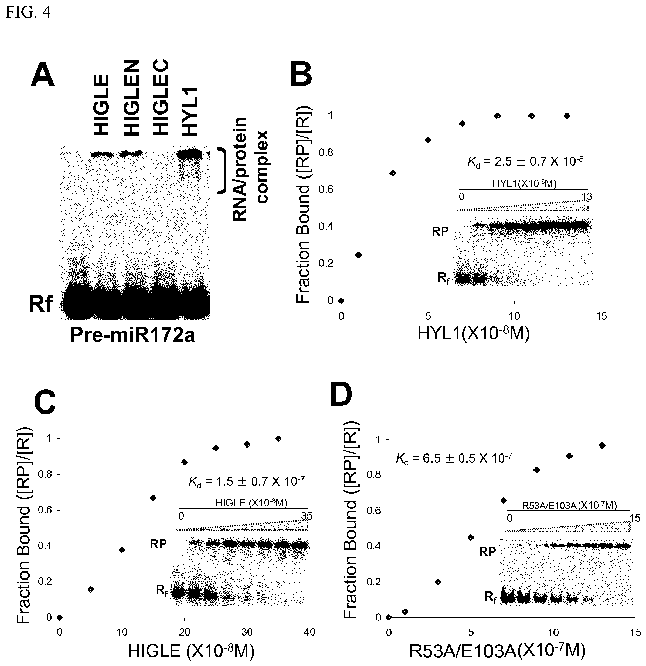

FIG. 4 shows results of performing a gel shift assay using pre-miR172a as a substrate. (A) shows results of adding full-length HIGLE, a domain-deleted fragment and HYL1. (B) shows a dissociation constant of HYL1 (5 × 10⁻⁹ M to 1.3 × 10⁻⁷ M) for pre-miR172a. (C) shows a dissociation constant of HIGLE (3 × 10⁻⁸ M to 3.5 × 10⁻⁷ M) for pre-miR172a. (D) shows a dissociation constant of R53A/E103A(9 × 10⁻⁸ M to 1.5 × 10⁻⁶ M) for pre-miR172a;

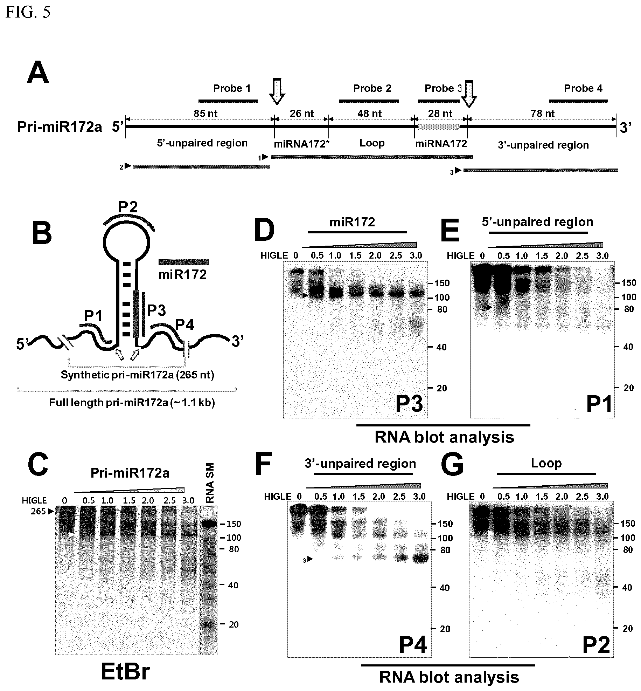

FIG. 5 shows synthetic pri-miR172a cleaved into pre-miRNA-type intermediate fragments by HIGLE. (A) schematically shows the synthetic pri-miR172a. (B) shows structured synthetic pri-miR172a. (C) shows analysis of processed fragments through EtBr staining. (D) shows pre-miRNA-type intermediates including miRNA confirmed through RNA blot analysis using P3 (miR172). (E) shows results of determining a 5'-single-stranded region using a PI (5'-flap) probe. (F) shows results of determining a 3'-single-stranded region using a P4 (3'-flap) probe. (G) shows results of determining an approximately 100 nt-long region of a pre-miRNA-type intermediate using a P2 probe corresponding to a loop structure;

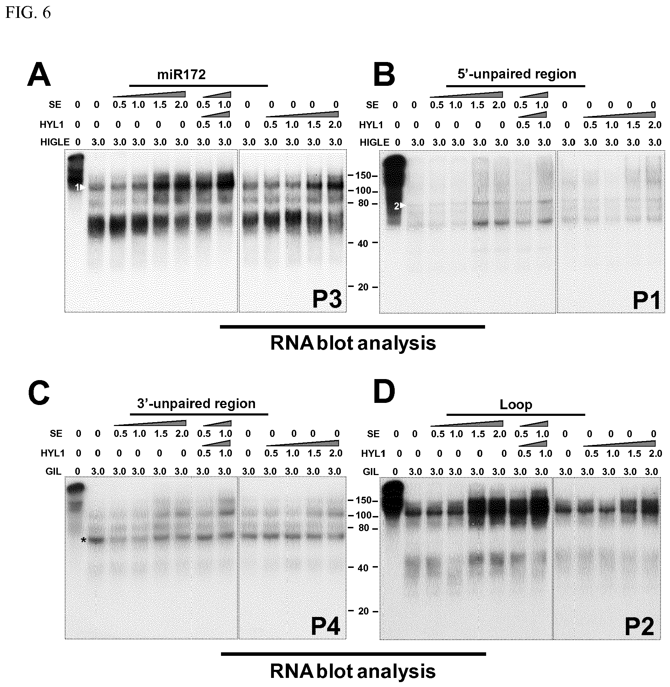

FIG. 6 shows results of analyzing a guiding effect of HYL1 and SE in HIGLE-mediated pri-miR172 processing. (A) shows increased accumulation of a pre-miR172a-type intermediate by SE or HYL1. (B) shows results of determining a 5'-single-stranded region using a P1 probe. (C) shows results of determining a 3'-single-stranded region using a P4 probe. (D) shows results of determining a loop structure of the pre-miRNA-type intermediate using a P2 probe;

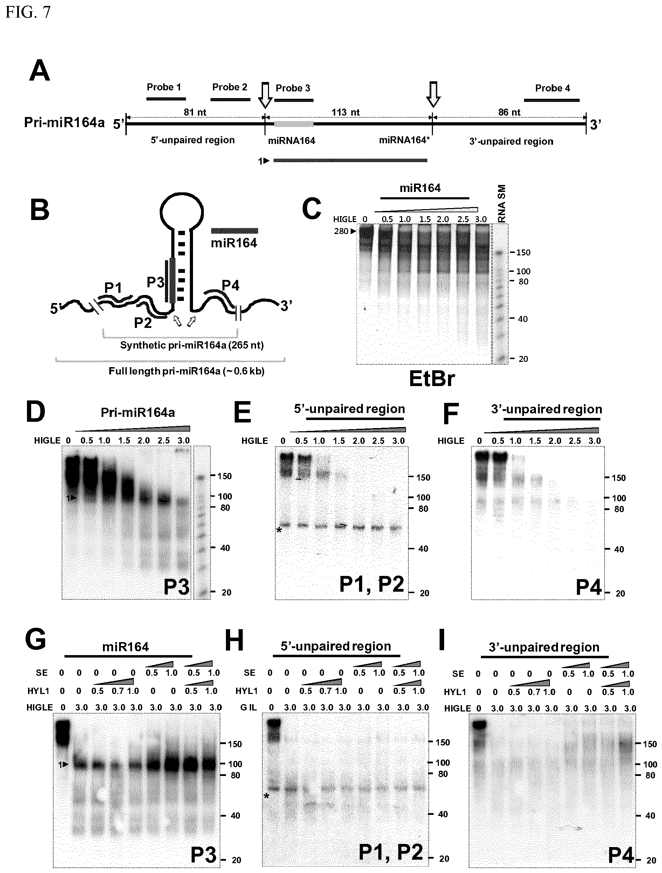

FIG. 7 shows synthetic pri-miR164a cleaved into pre-miR164-type intermediate fragments by HIGLE. (A) shows a configuration of the synthetic pri-miR164a used for processing analysis. (B) shows structured synthetic pri-miR164a. (C) shows analysis of processed fragments through EtBr staining. (D) shows results of determining a pre-miR164-type intermediate (approximately 100 nt) including miRNA through RNA blot analysis using P3 (miR164). (E) shows results of determining a 5'-single-stranded region using P1 and P2 probes. (F) shows results of determining a 3'-single-stranded region using a P4 probe. (G) shows increased accumulation of a pre-miR164a-type intermediate by SE or HYL1. (H) shows results of determining a 5'-single-stranded region using the P1 and P2 probes. (I) shows results of determining a 3'-single-stranded region using the P4 probe; and

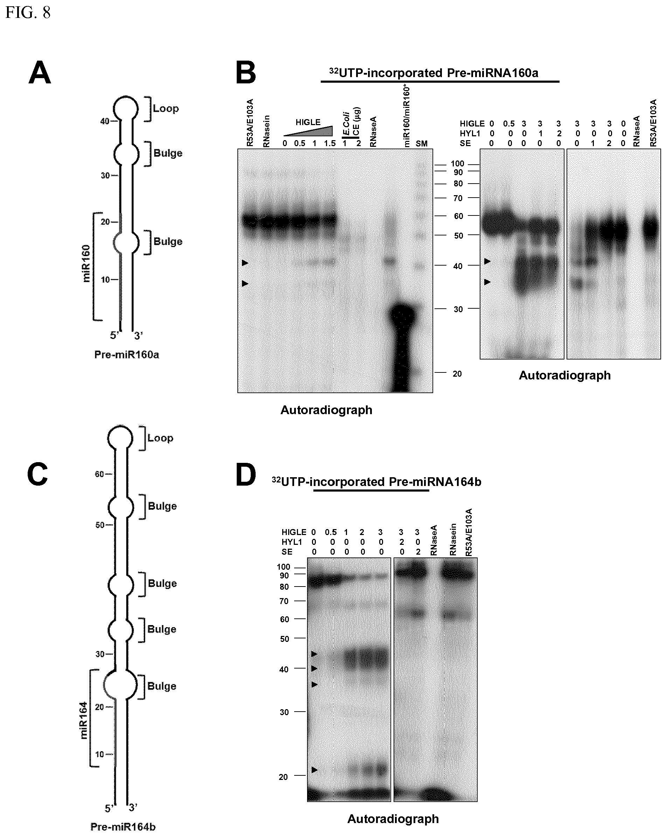

FIG. 8 shows analysis of cleavage activity of HIGLE using pre-miR160a and pre-miR164b. (A) schematically shows a pre-miR160a substrate. (B) shows the pre-miR160a substrate cleaved into two fragments by HIGLE. (C) schematically shows a pre-miR164b substrate. (D) shows the pre-miR164b substrate cleaved into four fragments by HIGLE.

### DETAILED DESCRIPTION OF EXEMPLARY EMBODIMENTS

Hereinafter, the present invention will be described with reference to the accompanying drawings. In the drawings, description of parts irrelevant to the detailed description are omitted in order to describe the present invention more clearly, and like numbers refer to like elements throughout the description of the figures.

Unless the context particularly indicates otherwise, it will be further understood that the terms "comprises," "comprising," "includes" and/or "including," when used herein, specify the presence of components and/or elements thereof, but do not preclude the presence or addition of other components and/or elements thereof.

Unless otherwise defined, molecular biology, microbiology, protein purification, protein engineering, and DNA sequencing may be performed using conventional techniques widely used in the recombinant DNA field within the scope of the skill of a person having ordinary skill in the art. The techniques are known to the person having ordinary skill in the art, and disclosed in many standardized textbooks and reference books.

Unless otherwise defined in this specification, all the technical and scientific terms used herein have the same meanings as what are generally understood by a person skilled in the related art to which the present invention belongs.

Various science dictionaries including the terms included in this specification are widely known and used in the related art. Although any method and material similar or equivalent to those disclosed in this specification are found to be used to put the present invention into practice or used for experiments, several methods and materials are disclosed in this specification. Since certain methodologies, protocols and reagents may be widely used depending on the contents used by those skilled in the art, the methodologies, protocols and reagents are not intended to limit the scope of the present invention.

As used in this specification, the singular forms "a," "an" and "the" are intended to include the plural forms as well, unless the context clearly indicates otherwise. Also, a nucleic acid sequence is written in a left-to-right direction, that is, a 5' to 3' direction, and an amino acid sequence is written in a left-to-right direction, that is, an amino-to-carboxyl direction, unless the context clearly indicates otherwise.

Hereinafter, the present invention will be described in further detail. According to an aspect of the present invention, there is provided the use of the isolated homing endonuclease (HIGLE) consisting of an amino acid sequence set forth in SEQ ID NO: 1 which (a) includes a GIY-YIG domain, (b) has a bifunctional nature based on RNA and DNA, and (c) exhibits construct-specific activity with respect to 3' and 5' overhangs.

According to one exemplary embodiment, the homing endonuclease may be isolated from *Arabidopsis thaliana.*

The term "endonuclease" refers to an enzyme that hydrolyzes a phosphodiester bond in a nucleic acid backbone, and the present invention provides a novel homing endonuclease isolated from *Arabidopsis thaliana.*

The present inventors have found a novel protein involved in the biogenesis of miRNA, and named the protein "HYL1 interacting GIY-YIG-like endonuclease (HIGLE)". In plants, a microprocessor may be generally composed of dicer-like protein 1 (DCL1), hyponastic leaf 1 (HYL1), and serrate (SE). The microprocessor may interact with at least 7 proteins during a miRNA maturation process.

The homing endonuclease (HIGLE) may include a GIY-YIG domain which is commonly found in some homing endonucleases. Although the homing endonuclease (HIGLE) is not related to an RNase type III family involved in the metabolism of sRNA, the homing endonuclease (HIGLE) may remove 5' and 3' overhangs to process pri-miRNA into a pre-miRNA-type intermediate.

The "homing endonuclease" belongs to a family of endonucleases that catalyze a gene conversion phenomenon in which an endonuclease-coding allele of a certain gene propagates into an endonuclease-deficient allele of the gene due to its biological functions.

For example, at least five types of the homing endonuclease are known, including i) a LAGLIDADG homing endonuclease, ii) a HNH homing endonuclease, iii) a His-Cys box homing endonuclease, iv) a GIY-YIG homing endonuclease, and v) a cyanobacterial homing endonuclease.

The isolated homing endonuclease (HIGLE) may include the GIY-YIG domain, and may have a bifunctional nature based on RNA and DNA.

The GIY-YIG domain has an α/β structure including a sequence of 90 to 100 amino acids, a space spanning from a -GIY- sequence to a -YIG- sequence may consist of approximately 10 to 11 amino acids to form a three-stranded antiparallel β-sheet structure. Residues important for the nucleic acid hydrolase activity may include glycine in a GIY-YIG motif, approximately 8 to 10 arginine residues downstream from the -GIY- sequence, a metal-binding glutamic acid residue such as glutamic acid at position 75 of I-TevI, and a conserved asparagine residue upstream from the metal-binding glutamic acid residue.

The present inventors have found through BLAST analysis that the GIY-YIG domain of the isolated homing endonuclease (HIGLE) belongs to a SLX1 cluster in a family of highly diversified GIY-YIG endonucleases.

In general, the GIY-YIG homing endonuclease may cleave a single-stranded 5'-flap of Y-shaped junction structure DNA and a loop structure of hairpin DNA, but the isolated endonuclease (HIGLE) may exhibit construct-specific cleavage activity with respect to 3' and 5' overhangs of a nucleic acid due to the activity of the GIY-YIG domain, and may have a bifunctional nature based on RNA and DNA.

Unlike conventional GIY-YIG homing endonucleases, the homing endonuclease (HIGLE) has sequence-non-specific activity as well as the cleavage activity against RNA, and thus may be effectively used to detect microRNA (miRNA) which has come into the spotlight as a factor important for diagnosis of diseases.

Specifically, in a hybridization method used to detect the miRNA, the complementarity between target nucleic acid sequences is judged to be positive when the miRNA perfectly match to the target nucleic acid sequences, but non-specific hybridization may be caused in spite of some mismatched bases, which leads to false positives.

In this case, since some non-complementary base pairs do not hybridize with each other, a bulge loop may be formed in some regions of a non-specifically hybridized duplex.

Since the homing endonuclease (HIGLE) is sequence-non-specific due to the activity of the GIY-YIG domain and has construct-specific activity, the homing endonuclease (HIGLE) may cleave a bulge loop including the 3' and 5' overhangs regardless of the sequences. Since the non-specifically hybridized duplex is cleaved due to the activity of the homing endonuclease (HIGLE), the false positives that may be caused due to mismatch upon miRNA detection may be excluded.

The homing endonuclease consists of an amino acid sequence set forth in SEQ ID NO: 1, and the GIY-YIG domain may consist of an amino acid sequence set forth in SEQ ID NO: 2.

The homing endonuclease and the GIY-YIG domain may include functional equivalents of a protein including the amino acid sequences of SEQ ID NOs: 1 and 2.

The term "functional equivalent" refers to a protein that has a sequence homology of at least 40% or more, preferably 80% or more, more preferably 90% or more, and most preferably 95% or more with respect to the amino acid sequence of SEQ ID NO: 1 as a result of addition, substitution or deletion of amino acids, and thus has substantially the same physiological activity as the amino acid sequence of SEQ ID NO: 1. The "substantially the same physiological activity" refers to a construct-specific cleavage activity caused due to structural and functional homology of the endonuclease. The "sequence homology" may be determined by comparing a comparison region with two optimally aligned sequences, and a portion of the nucleic acid sequence in the comparison region may be added or deleted.

An expression vector including a nucleic acid sequence encoding the homing endonuclease is also described.

The vector refers to a nucleic acid molecule used to deliver a nucleic acid fragment coupled thereto. A bacterium, a plasmid, a phage, a cosmid, an episome, a virus, and an insertable DNA fragment (i.e., a fragment insertable into the host cell genome by means of homologous recombination) may be used as the vector, but the present invention is not limited thereto. The plasmid is a type of the vector and refers to a circular double-stranded DNA loop that is capable of ligating an additional DNA fragment to an inner part thereof. Also, the virus vector may ligate additional DNA into the viral genome.

The expression vector refers to a vector that can instruct expression of a gene encoding an operably linked target protein. In general, since the expression vector in use of recombinant DNA technology is in the form of a plasmid, the terms "plasmid" and "vector" may be used interchangeably with each other. However, the expression vector may also include different types of expression vectors that perform the same function as the viral vector.

For example, the expression vector may include pET-3a-d, pET-9a-d, pET-11a-d, pET-12a-c, pET-14b, pET-15b, pET-16b, pET-17b, pET-17xb, pET-19b, pET-20b(+), pET-21a-d(+), pET-22b(+), pET-23a-d(+), pET-24a-d(+), pET-25b(+), pET-26b(+), pET-27b(+), pET-28a-c(+), pET-29a-c(+), pET-30a-c(+), pET-30 Ek/LIC, pET-30 Xa/LIC, pET-31b(+), pET-32a-c(+), pET-32 Ek/LIC, pET-32 Xa/LIC, pET-33b(+), pET-34b(+), pET-35b(+), pET-36b(+), pET-37b(+), pET-38b(+), pET-39b(+), pET-40b(+), pET-41a-c(+), pET-41 Ek/LIC, pET-42a-c(+), pET-43.1a-c(+), pET-43.1 Ek/LIC, pET-44a-c(+), pRSETA, pRSETB, pRSETC, pESC-HIS, pESC-LEU, pESC-TRP, pESC-URA, Gateway pYES-DEST52, pAO815, pGAPZ A, pGAPZ B, pGAPZ C, pGAPa A, pGAPa B, pGAPa C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPink™, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405, or pYJ406, but the present invention is not limited thereto.

Meanwhile, the expression vector may be introduced into a host cell, and thus the transformed host cell may produce the homing endonuclease through the introduced vector. In this case, the vector may include a promoter recognized by a host organism.

The promoter may be selected from the group consisting of SBE4, 3TP, PAI-1, p15, p21, CAGA12, hINS, A3, NFAT, NFKB, AP1, IFNG, IL4, IL17A, IL10, GPD, TEF, ADH, CYC, INU1, PGK1, PHO5, TRP1, GAL1, GAL10, GUT2, tac, T7, T5, nmt, fbp1, AOX1, AOX2, MOX1, and FMD1 promoters, and may vary in consideration of various parameters such as types of host cells, or expression conditions, etc.

The nucleic acid sequence encoding the homing endonuclease may be operably linked to the promoter sequence. The term "operably linked" means that one nucleic acid fragment is linked to another nucleic acid fragment so that the function and expression of the one nucleic acid fragment are affected by the other nucleic acid fragment. That is, a gene encoding the homing endonuclease may be operably linked to a promoter in a vector to regulate expression of the gene.

Meanwhile, the expression vector may further include an additional regulatory sequence. The regulatory sequence may include a Shine-Dalgano sequence of a replicase gene in phage MS-2, and a Shine-Dalgano sequence of bacteriophage lambda (λ) cII, but the present invention is not limited thereto.

Also, the expression vector may include a suitable marker gene required to select a transformed host cell. The marker gene may be an antibiotic-resistant gene or a fluorescent protein gene, and the antibiotic-resistant gene may be selected from the group consisting of a hygromycin-resistant gene, a kanamycin-resistant gene, a chloramphenicol-resistant gene, and a tetracycline-resistant gene, but the present invention is not limited thereto. The fluorescent protein gene may be selected from the group consisting of a yeast-enhanced green fluorescent protein (yEGFP) gene, a green fluorescent protein (GFP) gene, a blue fluorescent protein (BFP) gene, and a red fluorescent protein (RFP), but the present invention is not limited thereto.

A recombinant host cell that overexpresses an exogenous nucleic acid sequence encoding the homing endonuclease is also described.

The host cell may be metabolically engineered through transformation. The host cell refers to a cell that is easily transformed with an expression vector. In this case, types of host cells are not particularly limited as long as the host cells can be transformed by a genetic engineering method to effectively express a certain gene.

The host cell may be a microorganism, an animal cell, a plant cell, a culture cell derived from an animal, or a culture cell derived from a plant, but is not limited thereto. The proper host cell may be a naturally occurring or wild-type host cell, or a modified host cell. The wild-type host cell may be a host cell that is not genetically modified by a recombination method.

The term "metabolically engineered" or "metabolic engineering" may encompass reasonable pathway design and assembly of a biosynthetic gene, an operon-linked gene, and control elements of nucleic acid sequences thereof so as to produce desired metabolites such as alcohols or proteins in a microorganism.

The "metabolically engineered" may further include the optimization of metabolic flux through the control and optimization of transcription, translation, protein stability and protein functionality using gene manipulation and suitable culture conditions. The biosynthetic gene may be exogenous to the host, or may be heterologous to the host (for example, a microorganism) since the biosynthetic gene is modified with linkage with a heterologous expression control sequence in a mutagenetic, recombinant or endogenous host cell. The suitable culture conditions may include conditions such as pH of a culture medium, ionic strength, nutrient content, etc., culture conditions such as temperature, oxygen, carbon dioxide and nitrogen content, humidity, etc., and other conditions in which compounds may be produced by a metabolic action of the microorganism. The culture conditions suitable for microorganisms capable of functioning as the host cell are known in the related art.

Therefore, the "metabolically engineered" or "modified" host cell may be produced by introducing a genetic material into a selected host or parental microorganism to modify or alter cellular physiology and biochemistry. The parental microorganism may acquire new properties, for example, an ability to produce new intracellular metabolites or a larger amount of intracellular metabolites through the introduction of the genetic material.

For example, the introduction of the genetic material into the parental microorganism may lead to a new or modified ability to produce chemicals. The genetic material introduced into the parental microorganism includes a gene or fragments thereof which encode one or more enzymes involved in a biosynthetic pathway for producing the chemicals, and may include additional constitutional elements for expressing these genes, or regulating expression of the genes, for example, a promoter sequence.

The "altered host cell" refers to a genetically designed host cell. Here, a target protein may be expressed at a normal expression level, expressed at a higher level than the normal expression level, or expressed at a higher level than a level of expression of a target protein in an unaltered or wild-type host cell which grows under essentially the same growth conditions. The "modified host cell" refers to a wild-type or altered host cell genetically designed to overexpress a gene encoding the target protein. The modified host cell may express the target protein at a higher level than the wild-type or altered parental host cell.

Meanwhile, the "transformation" refers to a method of transporting the vector into a microorganism. When a cell to be transformed is a prokaryotic cell, the transformation may be performed using a CaCl₂ method (Cohen, S.N. et al., Proc. Natl. Acad. Sci. USA, 9:2110- 2114(1973)), a Hanahan method (Cohen, S.N. et al., Proc. Natl. Acad. Sci. USA, 9:2110-2114(1973); and Hanahan, D., J. Mol. Biol., 166:557-580(1983)), and an electroporation method (Dower, W.J. et al., Nucleic. Acids Res., 16:6127-6145(1988)), etc.

When the cell to be transformed is a eukaryotic cell, the transformation may be performed using a microinjection method (Capecchi, M.R., Cell, 22:479(1980)), a calcium phosphate precipitation method (Graham, F.L. et al., Virology, 52:456(1973)), an electroporation method (Neumann, E. et al., EMBO J., 1:841(1982)), a liposome-mediated transfection method (Wong, T.K. et al., Gene, 10:87(1980)), a DEAE-dextran treatment method (Gopal, Mol. Cell. Biol., 5:1188-1190(1985)), and gene bombardment (Yang et al., Proc. Natl. Acad. Sci., 87:9568-9572(1990)) etc., but is not limited thereto.

When fungi such as yeast is transformed, the transformation may be generally performed using transformation methods using lithium acetate (R.D. Gietz, Yeast 11, 355360(1995)) and heat shock (Keisuke Matsuura, Journal of Bioscience and Bioengineering, Vol. 100, 5; 538-544(2005)), an electroporation method (Nina Skolucka, Asian Pacific Journal of Tropical Biomedicine, 94-98(2011)), etc., but is not limited thereto.

A method of preparing a homing endonuclease is also described, which includes transforming a host cell with the expression vector; and culturing the host cell to express the homing endonuclease.

The transformed host cell may be cultured under batch, fed-batch or continuous fermentation conditions, and a fusion protein may be obtained from the cultured host cell since the host cell may express the fusion protein due to the transformation.

In this case, a conventional batch fermentation method may use a closed system, the culture medium is prepared prior to fermentation, an organism is inoculated in the medium, and the fermentation may occur without adding any components to the medium. In certain cases, the pH and oxygen content rather than the contents of the carbon source in a growth medium may vary during a batch method. The metabolites and cellular biomass in a batch system may constantly vary until the fermentation stops. In the batch system, the cells may progress throughout a high-growth log phase on a stationary member, and may finally reach a stationary phase in which the growth rate of the cells is reduced or the growth of the cells is stopped. During a typical period, the cells of the log phase may produce most proteins.

A modified standard batch system is a "fed-batch fermentation" system. In the system, nutrients (for example, a carbon source, a nitrogen source, O₂, and typically other nutrients) may be added when a concentration of these cultures drops to a subthreshold value. The fed-batch system may be useful when catabolite repression inhibits cell metabolism and a medium is desired to include a limited amount of nutrients. The measurement of the actual concentration of nutrients in the fed-batch system may be predicted based on a change in measurable factors such as pH, dissolved oxygen, a partial pressure of a waste gas such as CO₂. Batch fermentation and fed-batch fermentation are widely known as conventional systems in the related art.

The continuous fermentation is an open system in which a defined culture medium is continuously added to a bioreactor and the same amount of a conditioned medium is simultaneously removed during this procedure. In general, the continuous fermentation may sustain a constant high-density culture in which the cells grow in a log phase at the beginning. The continuous fermentation may be performed through manipulation of one factor or any number of factors which affect the cell growth or a concentration of the final product.

For example, limited nutrients such as a carbon source or a nitrogen source may be maintained at a constant rate, and all other parameters may be properly maintained. In other systems, a factor having a great influence on the growth may continuously vary while the cell concentration measured by the medium turbidity is constantly maintained. The continuous system may be required to maintain a certain growth condition. Therefore, the loss of cells which are removed with a medium may be balanced against a cell growth rate in the fermentation. As well as the technology of maximizing a product-forming rate, a method of maintaining nutrients and growth factors during the continuous fermentation process is known in the related art.

Hereinafter, the present invention will be described in further detail with reference to examples thereof. However, it will be obvious that the examples are not intended to limit the present invention.

### Experimental Example 1: Evolutionary characteristic test of HIGLE

The present inventors have isolated a clone encoding HIGLE through a yeast two-hybrid screening method. Sequence analysis showed that the replicated clone completely matched a 368 amino acid sequence of a protein encoded by the locus (At2g30350) . The structural correlation between HIGLE and GIY-YIG domains from other organisms was analyzed through protein identification.

BLAST analysis showed that the GIY-YIG domain of the HIGLE belongs to a SLX1 cluster in a family of highly diversified GIY-YIG endonucleases.

SLX1 present in yeast, a rat, and a human mainly includes two domains, that is, a GIY-YIG (URI) domain and a circular DNA-binding domain. The former is a construct-specific endonuclease that cleaves a single-stranded 5'-flap of Y-shaped junction structure DNA and a loop structure of hairpin DNA, and the latter may recognize a target DNA molecule.

Unlike the SLX1 protein from an animal, HIGLE does not include a circular DNA-binding domain and only includes the GIY-YIG domain. The GIY-YIG domain of HIGLE generally shares an identity of 46 to 83% with respect to other SLX1 proteins, and is most closely related with unidentified GIY-YIG domains from maize and rice (having an identity of approximately 83%).

On the other hand, the GIY-YIG domain of HIGLE has a limited homology with respect to SLX1 proteins from a human (approximately 54%) and yeast (approximately 46%).

Although the overall structure of HIGLE is slightly different from other SLX1 proteins, the high similarity of the GIY-YIG domain suggests that HIGLE can function as a construct-specific homing endonuclease.

### Experimental Example 2: Test on formation of complex of HIGLE, HYL1 and SE

To analyze whether HIGLE directly forms a protein complex with HYL1, a pull-down assay was performed *in vitro.*

In the analysis, a variant in which a HIGLE protein, the N-terminus, and the C-terminus were deleted was expressed as a protein fused with a maltose-binding protein (MBP), and three sites of the N-terminus and the C-terminus were HA-tagged. The fused protein was purified by MBP-affinity chromatography.

Similarly, HYL1 and a deletion variant including RNA-binding domain 1 and RNA-binding domain 2 at the N-terminus were expressed, and purified using histone-affinity chromatography (FIG. 1C).

An MBP-fused HIGLE-HA protein was mixed with HIS-fused HYL1 and a deletion variant protein, and then subjected to pull-down analysis in an amylose resin.

MBP-HIGLE-3HA pulled the HYL1 and RBD1+2 domains down, but did not pull the separate RNA-binding domain down. On the other hand, when the MBP protein was used as the negative control, neither the HYL1 nor deletion variant was recovered (FIG. 1D).

On the other hand, GST-fused HYL1 was used to determine a HIGLE interaction domain. GST-HYL1 bound to an N-terminal region of HIGLE (FIG. IE).

The results show that HIGLE can directly interact with the N-terminal region of HYL1 via the GIY-YIG domain. Also, it was confirmed that HIGLE was able to interact with another guiding protein, that is, serrate (SE). The GST-fused SE-deletion variant was prepared in the same manner as described above.

Both N-terminal and middle regions of SE interacted with HIGLE and the GIY-YIG domain of HIGLE (FIG. 1D). In both cases, GST alone did not interact with the HIGLE or deletion variant, suggesting the specificity of a binding assay.

That is, the results suggest that the GIY-YIG domain of HIGLE can mediate direct binding to the N-terminus of HYL1 and the N-terminal and middle regions of SE. Since the protein binding between HIGLE, HYL1 and SE suggested an *in vivo* mutual relationship, a co-immunoprecipitation method (co-IP) was performed using a WT/35S-HIGLE-6Myc or hyl1-2/35S-HYL1-6Myc gene-transplanted plant. Precipitation of an anti-MYC antibody (immuno-complex) was determined using anti-HIGLE, anti-HYLl, and anti-SE antibodies. HYL1-6myc was able to be co-immunoprecipitated with endogenous HIGLE.

A knocked-down transgenic plant line (#3) was used as the negative control to check binding specificity (FIG. 1D).

Similarly, when HIGLE-6Myc was co-immunoprecipitated with an α-Myc antibody, the endogenous SE was detected in the precipitated complex. A knocked-down transgenic plant line (#18) was used as the negative control to measure an interaction between SE and HIGLE (FIG. 1D).

The present inventors have found that the nuclear localization of HYL1 is already well known, and assumed that HIGLE is co-localized with HYL1 in the nucleus since the HIGLE emits a nuclear localization signal at the N-terminal and C-terminal regions.

HIGLE-RFP was mainly localized in the nuclei of a transplanted seedling of the WT/35S-HIGLE-RFP plant.

Also, a double transgenic plant line WT/35S-HYL1-CFP/35S-HIGLE-YFP was used to visualize the interaction between HIGLE and HYL1. HIGLE-YFP was expressed with HYL1-CFP at a high level in the nuclei, and HIGLE and HYL1 were localized in the nuclei at the same time (FIG. 2A).

Fluorescence resonance energy transfer (FRET) analysis was performed to evaluate physical proximity between HIGLE and HYL1.

Effective energy transfer was observed using HYL1-CFP and HIGLE-YFP as a donor-acceptor pair, suggesting that the protein linked two fluorogens so that the two fluorogens were maintained at a close distance sufficient for the energy transfer (less than 100 Å).

The proteins were not divided into speckles or dicing bodies, but rather the interaction occurred throughout the nuclei, which was assumed to arise from the fact that that the proteins were expressed at a high level under the control of a 35S promoter.

That is, the results suggest that HIGLE and SE interact with each other *in vivo,* and HIGLE forms a complex with HYL1 and SE.

### Experimental Example 3: Test on RNA and DNA construct-specific activity of HIGLE

SLX1 is a type of construct-specific homing endonuclease that may remove a single strand from a DNA substrate, which has a double-stranded (DS)/single stranded (SS) junction structure, through the 3'-to-5' nuclease activity.

When DNA with a hairpin (stem-loop) structure and DNA with a Y-shaped structure well known as the DS/SS junction structure were applied, the yeast SLX1 cleaves a 3'-to-5' single strand proximate to a junction region of the Y-shaped DNA.

To check whether HIGLE had a junction structure-specific nuclease activity, the present inventors have performed processing analysis using the Y-shaped DNA as a substrate.

For the processing analysis, Y-shaped DNA substrates represented by DY1 and DY2 were prepared.

The Y-shaped DNA substrates had the same sequence, but were labeled at different positions. For one Y-shaped DNA substrate, a double-stranded 5'-terminal region (DY1) was labeled with radioactive phosphate, and, for the other Y-shaped DNA substrate, a single-stranded 5'-terminal region (DY2) was labeled with radioactive phosphate.

Similar to the yeast SLX1 activity, HIGLE effectively cleaved 3'-to-5' overhangs of the junction of DY2. On the other hand, processing of a 5'-to-3' overhang strand of DY1 was delayed (FIG. 3A).

The results suggest that HIGLE has construct-specific DNase activity.

Although the DNase activity of HIGLE was determined, the correlation between the DNase activity of HIGLE and HYL1-interaction characteristics was not determined.

Recent phylogenetic studies proposed that the GIY-YIG protein and RNase HI evolved from a RNA-binding protein. Also, many studies reported that some homing endonucleases have a bifunctional nature of acting on both DNA and RNA.

To determine whether HIGLE had the bifunctional nature, the present inventors replaced the Y-shaped DNA with RNA, and named the resulting proteins "RY1" and "RY2."

Based on the same experimental results, it was confirmed that HIGLE effectively cleaved 3'-to-5' single-stranded RY2, and thus had the bifunctional nature.

The 5'-to-3' strand of RY1 was removed by HIGLE, and the HIGLE had somewhat excellent activity, compared to the DNase activity (FIG. 3B).

The construct-specific RNase activity of HIGLE was confirmed using single-stranded, non-structured RNA as the control (FIG. 3C).

Also, the present inventors have expressed HIGLE in *E. coli* including many endogenous endonucleases, and purified the expressed HIGLE. In this case, point mutations were introduced to arginine (R53A) and glutamic acid (E103A) residues in the GIY-YIG domain to check the nuclease activity by HIGLE. The same residues of yeast SLX1 and *E. coli* UvrC proteins are essential for the 3'-to-5' nuclease activity.

The N-terminal region of HIGLE and the whole sequence of HIGLE were successfully removed from the Y-shaped RNA substrate as a single strand, but the 3'-to-5' single strand was not removed in the double-point mutants and the C-terminal fragment of HIGLE (FIG. 3D).

The results suggest that HIGLE is the first bifunctional nuclease belonging to the family of GIY-YIG homing endonucleases.

### Experimental Example 4: Test on pre-miRNA-binding activity of HIGLE

HYL1 is a double-stranded RNA-binding protein that recognizes a fold-back stem structure of pri-miRNA.

Also, the RNA-binding domain of SE is important for precise processing of pri-miRNA.

DCL1 directly interacts with pri-miRNA through the RNA-binding domain, and may process the pri-miRNA into mature miRNA. The key microprocessing protein shares common characteristics with the double-stranded RNA-binding activity.

Therefore, the present inventors performed a gel shift assay (EMSA) using pre-miR172a as a substrate, and tested the double-stranded RNA-binding activities of HIGLE and deletion variants.

HIGLE was able to directly interact with pre-miR172a through the GIY-YIG domain (FIG. 4A).

The present inventors have measured the specific binding affinity of HIGLE to evaluate the binding of pre-miR172a/HIGLE.

The dissociation constant of HIGLE was 1.5 ± 0.7 × 10⁻⁷, which was six fold lower than that of HYL1 (2.5 ± 0.7 × 10⁻⁸), but this value can be generally assessed as a strong protein-RNA interaction (FIGS. 4B and 4C).

The results suggest that the GIY-YIG domain of HIGLE is sufficient to interact with the fold-back stem structure of pri-miRNA. Also, the double-point mutant (R53A/E103A) of HIGLE had an affinity to the pre-miR172a which was four fold lower than HIGLE (FIG. 4D).

That is, it can be evaluated that the RNase activity lowered due to the double point mutation was based on a decrease in binding affinity and catalytic activity.

### Experimental Example 5: Test on 5' and 3' overhang cleavage activity of HIGLE

Recent studies reported that a self-complementary stem structure of pri-miRNA is an important determinant when pri-miRNA is processed into mature miRNA. The studies first proposed a structural determinant of a miRNA precursor for miRNA biogenesis in plants.

However, since the length and nature of the fold-back stem structure have a wide range of diversity, a common structural determinant for pri-miRNA processing in the plants was not completely identified.

Based on the studies, the present inventors have assumed that HIGLE recognizes structural characteristics of pri-miRNA, for example, a typical DS/SS junction structure (FIGS. 5A and 5B; yellow arrows) and a bulge of a fold-back stem, and proved this assumption.

Since it was difficult to handle intact pri-miR172a under experimental conditions due to the length of the intact pri-miR172a, a pri-miR172a substrate including deleted 5' and 3' overhangs was synthesized.

The synthesized pri-miR172a was composed of 5'-SS (85nt), 3'-SS (78nt), and a fold-back stem (102nt), and had the same function as artificial pri-miR172a reported in the prior art (FIG. 5A).

When the substrate was cultured with an increasing concentration (0 to 3.0 × 10⁻⁸ g) of HIGLE, HIGLE cleaved the synthesized pri-miR172a into fragments having various sizes (50 nt to 150 nt) (FIG. 5C).

To determine which fragment corresponds to pre-miR172a (a fold-back stem, FIG. 5B) or mature miR172, RNA blot analysis was performed using a probe specific to each of a 5'-unpaired region (5'-flap), a loop, a miR172 region, and a 3'-unpaired region (3'-flap) represented by P1, P2, P3, and P4, respectively (FIGS. 6A and 6B).

Among the processed fragments, a fragment including the miR172 region (approximately 100 nt) was detected when the P3 probe (miR172) was used (arrow 1). An additional fragment (approximately 60 nt) was detected together with the miR172 region at a concentration of 3.0 × 10⁻⁸ g (FIG. 6D).

The approximately 100 nt-long and approximately 60 nt-long fragments were assumed to be a pre-miR172a-type intermediate, and a further truncated derivative.

To determine whether the approximately 100 nt-long fragment is pre-miR172a having no 5'- and 3'-flaps, a cell membrane was re-hybridized with P1 (5'-flap) or P4 (3'-flap). The 5'- and 3'-flaps were not sufficiently detected in the vicinity of 100 nt, which suggests that the approximately 100 nt-long fragment may be a fold-back stem of the synthetic pri-miR172a in the absence of both the flaps (FIGS. 5B, 5E and 5F).

Also, it was determined whether the approximately 100 nt-long intermediate had both a loop structure and a miR172 region using a P2 probe (FIG. 5G).

The results suggest that HIGLE may cleave the DS/SS junction structure of pri-miR172a, and may produce the pre-miR172a-type intermediate (FIGS. 5A and 5B; yellow arrows).

The present inventors have replicated and sequenced the fragments cleaved through the processing analysis, and verified that the intermediate was actually a fold-back stem of the pri-miR172a (FIG. 5A; red lines).

Although a single-stranded nucleic acid was an improper substrate for HIGLE, the fragmented 5'-flap was hardly detected at an expected size (85 nt) (FIG. 5E; arrow), but the 3'-flap fragment was clearly observed at an expected size (78 nt) (FIG. 5F).

The results did not completely correspond with an actual state of the substrate, but an M-fold computational prediction program predicted that the 5'-flap region forms a small fold-back stem and a bulge.

Therefore, the present inventors have assumed that the 5'-flap region may be degraded into small undetectable fragments by HIGLE.

It has been reported that HYL1 and SE performed an important function together for precise processing of the pri-miRNA by DCL1.

Therefore, the present inventors have performed processing analysis while maintaining HIGLE at a constant concentration (3.0 × 10⁻⁸ g) and increasing a concentration of a HYL1 or SE protein.

As the SE or HYL1 was added, production of the approximately 100 nt-long fragment increased, and thus the approximately 60 nt-long fragment decreased (FIG. 6A). The approximately 100 nt-long intermediate fragment was more effectively accumulated by SE (4 fold), compared to that of HYL1 (2 fold).

When both the HYL1 and SE were added, the approximately 100 nt-long fragment increased approximately 6 fold or more, compared to the control (only HIGLE added), suggesting an induction action in which HYL1 and SE were involved for the pri-miRNA processing of HIGLE (FIG. 6A). That is, the HYL1 and SE were able to protect the fold-back stem from the activity of HIGLE.

The characteristics of the pre-miR172a-type intermediate were identified through re-hybridization of the P1, P2 and P4 probes with the cell membrane. Since the 5'-flap and 3'-flap regions were not generally found in the intermediate (approximately 100 nt) (FIGS. 6B and 6C), and signals from the P4 probe were mainly detected in the same region of the intermediate, it was confirmed that the HYL1 and SE promoted formation of the intermediates (FIG. 6D).

Also, to verify the RNase activity of HIGLE for processing of pri-miRNA, *in vitro* processing analysis was performed using the synthesized pri-miR164a.

The synthesized pri-miR164a substrate is composed of 5'-flap (81 nt), and a fold-back stem (FIGS. 7A and 7B). When the substrate was cultured with HIGLE, the substrate was processed into fragments with various sizes (100 nt to 250 nt) (FIG. 7C).

To check the pre-miR164a-type intermediate of the fragment, RNA blot analysis was performed using probes corresponding to 5'-flap (PI and P2), a miR164 region (P3), and a 3'-flap region (P4) (FIG. 7A, 7B).

When the P3 probe was used, the 100 nt-long fragment including miR164 was formed with an increasing concentration of HIGLE (FIG. 7D).

To verify whether the approximately 100 nt-long fragment is the pre-miR164a-type intermediate, P1 and P2 or P4 were re-hybridized with the same cell membrane. Signals for the 5'-flap and 3'-flap regions were not detected in the approximately 100 nt-long fragment, and the results strongly supported the role of HIGLE in formation of the pre-miRNA-type intermediate (FIGS. 7E and 7F).

In this case, the pri-miR164a and pri-miR172a had slightly different cleavage patterns. For example, the RNA blot analysis showed that both the 5'-flap and 3'-flap were not detected at expected positions (81 nt and 86 nt) (FIGS. 7E and 7F).

Also, the guiding effects of HYL1 and SE were analyzed for the processing of the synthetic pri-miR164a.

Unlike the pri-miR172a, HYL1 did not effectively promote formation of the pre-miR164a-type intermediate. Further, a level of the pre-miR164a-type intermediate markedly increased in the presence of SE (FIG. 7G).

The characteristics of the pre-miR164a-type intermediate were verified using the P1, P2, and P4 probes (FIGS. 7H and 7I). From the control experiment in which contamination by *E. coli* endonucleases was excluded, it was confirmed that the HYL1 and SE were unable to process the pri-miR164a.

A difference in processing patterns of the synthetic pri-miR172a and pri-miR164a may be based on a difference in secondary structure or unknown factors. In the processing by HIGLE, it was assumed that HYL1 or SE protected the fold-back stem structure (a local Y-shaped structure) including a bulge from the activity of HIGLE (FIGS. 5 and 7).

Also, an experiment was further performed using an additional pre-miRNA-type substrate (including radioactively labeled UTP), pre-miR160a, and pre-miR164b. The former includes two bulges in a fold-back stem structure thereof, and the latter includes four bulges (FIGS. 8A and 8C).

The fold-back stem structure that was able to be processed into smaller fragments was observed with an increasing concentration (0 to 3.0 × 10⁻⁸ g) of HIGLE.

Two fragments (approximately 35 nt and approximately 40 nt) were generated from the pre-miR160a due to the RNase activity, and five fragments (four fragments spanning from 35 nt to 50 nt, and a single fragment in the vicinity of 20 nt) were generated from the pre-miR164b (FIGS. 8B and 8D). Considering the common characteristics of the homing endonucleases, the distinct patterns of both the fold-back stem substrates were analyzed to be based on a difference in the number of bulges (FIGS. 8B and 8C).

Also, the RNase activity of HIGLE was blocked by HYL1 or SE (FIGS. 8B and 8D), and HYL1 and SE were able to play an important role in protecting the fold-back stem structure. That is, the results suggest that HIGLE is a novel type of RNase that cleaves both 3' and 5' overhangs of the pri-miRNA, and may produce pre-miRNA-type intermediates during the induction of HYL1 and SE.

In the case of miRNA, primary miRNA (pri-miRNA) was generally processed into precursor miRNA (pre-miRNA) in plants, and a processing protein consisting of dicer-like protein 1 (DCL1), hyponastic leaf 1 (HYL1), and serrate (SE) participates in this process.

As an RNase type III slicer that cleaves the 5' and 3' overhang of pri-miRNA, DCL1 generates pre-miRNA, cleaves a loop region of the pre-miRNA, and eventually releases miRNA.

In a two-step cleavage process, the proteins HYL1 and SE were essentially involved in precise processing of miRNA. As a double-stranded RNA-binding protein, HYL1 binds to duplex miRNA/miRNA*.

HYL1 may directly interact with DCL1 and SE through RNA-binding domain 2 (RBD2), and mediate precise pri-miRNA processing. As a zinc-finger protein that serves to improve processing precision of pri-miRNA, SE forms a key microprocessor with DCL1 and HYL1.

The processed duplex miRNA/miRNA* is further modified by HUA enhancer 1 (HEN1). HEN1 adds a methyl group to a 2'-OH position of each of the duplexes miRNA/miRNA* and siRNA/siRNA* to prevent degradation of polyuridine through conjugation. The methylated miRNA/miRNA* duplex is transported into the cytoplasm by HASTY.

The miRNA is involved in a number of physiological, developmental, metabolic processes in eukaryotes. The primary miRNA (pri-miRNA) may be processed into 21 to 22 nt-long mature miRNA by the highly organized complex known as a microprocessor.

HYL1 and SE directly interacted with HIGLE to guide cleavage of a unpaired single-stranded region and protect a fold-back stem region of the pri-miRNA. Therefore, the results suggest that the HIGLE is an RNase that assists or supports pri-miRNA processing in plants.

The homing endonuclease described herein has a bifunctional nature with respect to DNA and RNA and serves to cleave single-stranded 3' and 5' overhangs, and thus can effectively eliminate false positives during nucleic acid detection.
## Claims

1. Use of an isolated homing endonuclease (HIGLE) consisting of the amino acid sequence set forth in SEQ ID NO: 1 which (a) comprises a GIY-YIG domain, (b) has a bifunctional nature based on RNA and DNA, and (c) exhibits construct-specific activity with respect to 3' and 5' overhangs, in the detection of microRNA (miRNA).

2. The use according to claim 1, wherein the homing endonuclease is isolated from *Arabidopsis thaliana.*

3. The use according to claim 1, wherein the GIY-YIG domain consists of the amino acid sequence set forth in SEQ ID NO: 2.

4. A method of cleaving a nucleic acid, comprising:
treating a nucleic acid with the homing endonuclease as defined in any one of claim 1 to 3.

5. The method of claim 4, wherein the nucleic acid is a double-stranded nucleic acid comprising 3' and 5' overhangs.

6. The method of claim 5, wherein the double-stranded nucleic acid has a bulge structure formed by non-complementary base pairing between base sequences in regions other than the 3' and 5' overhangs.

## Patentansprüche

1. Verwendung einer isolierten Homing-Endonuklease (HIGLE) bestehend aus der Aminosäuresequenz wie sie in SEQ ID NO: 1 angegeben ist, die (a) eine GIY-YIG Domäne umfasst, (b) eine auf RNA und DNA basierende bifunktionelle Natur, und (c) eine konstruktspezifische Aktivität in Bezug auf 3'- und 5'-Überhänge aufweist, im Nachweis von microRNA (miRNA).

2. Verwendung nach Anspruch 1, wobei die Homing-Endonuklease aus *Arabidopsis thaliana* isoliert ist.

3. Verwendung nach Anspruch 1, wobei die GIY-YIG Domäne aus der Aminosäuresequenz besteht, wie sie in SEQ ID NO: 2 angegeben ist.

4. Verfahren zum Spalten einer Nukleinsäure, umfassend:
Behandeln einer Nukleinsäure mit der Homing-Endonuklease wie sie in einem der Ansprüche 1 bis 3 definiert wurde.

5. Verfahren nach Anspruch 4, wobei die Nukleinsäure eine doppelsträngige Nukleinsäure ist, welche 3'- und 5'-Überhänge umfasst.

6. Verfahren nach Anspruch 5, wobei die doppelsträngige Nukleinsäure eine Ausbuchtungsstruktur aufweist, welche durch nicht komplementäre Basenpaarung zwischen Basensequenzen in anderen Regionen als den 3'- und 5'-Überhängen gebildet sind.

## Revendications

1. Utilisation d'une endonucléase de homing isolée (HIGLE) composée de la séquence d'acides aminés définie par la SEQ ID N°l, qui (a) comprend un domaine GIY-YIG, (b) a une nature bifonctionnelle basée sur l'ARN ou l'ADN, et (c) présente une activité spécifique de construction par rapport aux extrémités 3' et 5', dans la détection de micro-ARN (miRNA).

2. Utilisation selon la revendication 1, dans laquelle l'endonucléase de homing est isolée à partir d'*Arabidopsis thaliana.*

3. Utilisation selon la revendication 1, dans laquelle le domaine GIY-YIG est composé de la séquence d'acides aminées définie par la SEQ ID N°2.

4. Procédé de clivage d'un acide nucléique, consistant à :
traiter un acide nucléique avec l'endonucléase de homing selon une quelconque des revendications 1 à 3.

5. Procédé selon la revendication 4, dans lequel l'acide nucléique est un acide nucléique double brin comprenant des extrémités 3' et 5'.

6. Procédé selon la revendication 5, dans lequel l'acide nucléique double brin a une structure de bombement formée par un appariement de base non complémentaire entre des séquences de base dans des zones différentes des extrémités 3' et 5'.


<110> YONSEI UNIVERSITY
<120> NOVEL HOMING ENDONUCLEASE FROM ARABIDOPSIS THALIANA
<130> 16OP12109US
<160> 2
<170> KoPatentIn 3.0
<210> 1
   <211> 368
   <212> PRT
   <213> Arabidopsis thaliana
<400> 1
   Met Arg Glu Lys Arg Gly Asn Arg Lys Ala Leu Asp Pro Val Gly Glu
   1               5                   10                  15
   Asp Gly Val Thr Gly Lys Asp Gly Lys Gly Phe Phe Ala Cys Tyr Leu
               20                  25                  30
   Leu Thr Ser Leu Ser Pro Arg His Lys Gly Gln Thr Tyr Ile Gly Phe
           35                  40                  45
   Thr Val Asn Pro Arg Arg Arg Ile Arg Gln His Asn Gly Glu Ile Thr
       50                  55                  60
   Ser Gly Ala Trp Arg Thr Lys Lys Lys Arg Pro Trp Glu Met Val Leu
   65                  70                  75                  80
   Cys Ile Tyr Gly Phe Pro Thr Asn Val Ser Ala Leu Gln Phe Glu Trp
                   85                  90                  95
   Ala Trp Gln His Pro Arg Glu Ser Val Ala Val Arg Glu Ala Ala Ala
               100                 105                 110
   Ala Phe Lys Ser Phe Ser Gly Val Ala Ser Lys Ile Lys Leu Val Tyr
           115                 120                 125
   Thr Met Leu Asn Leu Pro Ala Trp Asn Ser Leu Asn Leu Thr Val Asn
       130                 135                 140
   Tyr Phe Ser Ser Lys Tyr Ala His His Gly Gly Lys Ser Pro Ser Leu
   145                 150                 155                 160
   Pro Leu His Met Lys Val Gln Val Cys Ala Met Glu Asp Leu Gln Tyr
                   165                 170                 175
   Phe Thr Lys Val Asp Asp Ser Ser Gln Pro Glu Asp Glu Glu Ser Pro
               180                 185                 190
   Glu Val Asn Glu Glu Asp Asp Asp Asp Asp Asp Asp Asp Ser Ser Asn
           195                 200                 205
   Leu Ser Gln Pro Gly Asn Ser Asn Thr Ser Ser Ser Asp Asp Arg His
       210                 215                 220
   Phe Glu Lys Ala Lys Glu Pro Val Thr Val Phe Asp Glu Glu Asp Arg
   225                 230                 235                 240
   Leu Ala Asn Phe Ser Gly Phe Gly Leu Leu Asp Glu Glu Glu Thr Val
                   245                 250                 255
   Glu Asp Glu Val Ser His Ile Thr Val Gly Ser Ile Arg Ala Thr Glu
               260                 265                 270
   Lys Glu Pro Glu Thr Val Phe Asn Asp Arg Leu Ala Ser Phe Thr Cys
                   275                 280                 285
   Phe Gly Leu Val Glu Ile Val Glu Asp Glu Val Ser His Gly Thr Ile
       290                 295                 300
   Gly Ser Thr Glu Ala Met Glu Lys Glu Cys Arg Lys Arg Arg Asn His
   305                 310                 315                 320
   Ile Thr Ser Thr Thr Thr Glu Val Asp Val Glu Val Ile Asp Leu Met
                   325                 330                 335
   Thr Pro Ser Pro Ser Cys Arg Ala Gly Ser Ser Met Lys Arg Arg Arg
               340                 345                 350
   Val Ser Glu Phe Ile Asp Leu Thr Met Ser Pro Asn Phe Ile Glu Ser
           355                 360                 365
<210> 2
   <211> 87
   <212> PRT
   <213> Arabidopsis thaliana
<400> 2
   Phe Phe Ala Cys Tyr Leu Leu Thr Ser Leu Ser Pro Arg His Lys Gly
   1               5                   10                  15
   Gln Thr Tyr Ile Gly Phe Thr Val Asn Pro Arg Arg Arg Ile Arg Gln
               20                  25                  30
   His Asn Gly Glu Ile Thr Ser Gly Ala Trp Arg Thr Lys Lys Lys Arg
           35                  40                  45
   Pro Trp Glu Met Val Leu Cys Ile Tyr Gly Phe Pro Thr Asn Val Ser
       50                  55                  60
   Ala Leu Gln Phe Glu Trp Ala Trp Gln His Pro Arg Glu Ser Val Ala
   65                  70                  75                  80
   Val Arg Glu Ala Ala Ala Ala
                   85